# EUROPEAN PATENT SPECIFICATION

(11) **EP 3 979 959 B1**
(45) Date of publication and mention of the grant of the patent: **14.01.2026**
(21) Application number: 20746386.0
(22) Date of filing: 29.05.2020
(51) Int. Cl.: A61F 5/441, A61F 5/445

(54) **OSTOMY POUCH WITH FILTER PATH AND FILTER**
OSTOMIEBEUTEL MIT FILTERBAHN UND FILTER
POCHE DE STOMIE AVEC TRAJET DE FILTRE ET FILTRE

(30) Priority: 04.06.2019 US 201962857009 P
(43) Date of publication of application: 13.04.2022
(73) Proprietor: Hollister Incorporated, Libertyville IL 60048 (US)
(72) Inventor: BOTTEN, Ronald, S., Libertyville, IL 60048 (US); CZAPLEWSKI, Gregory, J., Libertyville, IL 60048 (US)
(74) Representative: FRKelly
(86) International application number: PCT/US2020/035257
(87) International publication number: WO 2020/247268

(56) References cited:
- WO-A1-2007/000167
- WO-A1-2011/150937
- WO-A1-2019/058126
- US-A- 3 952 727
- US-A1- 2005 075 616

## Description

### BACKGROUND

The following description relates to an ostomy appliance, for example, an ostomy appliance having a filter path formed upstream from a filter assembly.

An ostomy bag or pouch includes an inlet configured to receive liquid, semisolid or solid bodily waste discharged from a stoma for collection within the pouch. A known pouch also includes a filter assembly to facilitate odor filtering and egress of gas from the pouch. However, in some instances, liquid, semisolid or solid contents (i.e., bodily waste) may flow to and block the filter assembly, thereby restricting egress of gas through the filter assembly. This may lead to a build up of gas pressure and undesirable inflation of the pouch.

A known ostomy pouch has been manufactured to add hydrophobic or oleophobic coatings or membranes to protect filter media of the filter assembly, in an effort to prevent clogging or fouling of the filter assembly by solid, semisolid or liquid pouch contents. However, such membranes are typically expensive and require additional materials and manufacturing steps.

Another known ostomy pouch, of the type described in US 5,690,623, adds a filter protecting panel between panel walls of the ostomy pouch. However, such an ostomy pouch requires additional materials (i.e., the filter protecting panel) and manufacturing steps.

Still another known ostomy pouch, of the type described in US 7,789,866, provides a filter assembly having a gas filter and a pre-filter for preventing or delaying solid/semisolid matter and liquid from reaching the gas filter. The pre-filter is at least substantially flat and comprises a number of constrictions, such as ribs extending along and between two inner surface parts of the channel. These constrictions/ribs form narrower and wider passages where the gas may more quickly pass a narrow passage and where the wider passages tend to receive and hold the liquid, solid matter and semisolid matter. However, a gas flow path may vary in width with gas pressure, and thus, it can be difficult to reliably restrict flow of solid, semisolid or liquid materials in the gas flow path.

Yet another known ostomy pouch, of the type described in WO 2017/192685, commonly assigned with the present application, includes a tortuous path formed in part by internal wall structures and a one-way valve. However, it may be difficult to control the materials entering the tortuous path.

WO2007000167A1 discloses an ostomy bag with a filter assembly having a gas filter and a pre-filter for preventing or delaying solid/semisolid matter and liquid from reaching the gas filter. The pre-filter is at least substantially flat and comprises a number of constrictions, such as ribs extending along and between two inner surface parts of the channel. These constrictions/ribs form narrower and wider passages where the gas may more quickly pass a narrow passage and where the wider passages tend to receive and hold the liquid, solid matter and semisolid matter. At least a part of the constrictions may be provided in a random pattern.

US 2005/075616 discloses an ostomy bag comprising (a) a main chamber with an upper extremity: (b) at least one multiple-use latent tube co-formed with said main chamber, said multiple-use latent tube having a proximal end firmly attached to, opening to and capable of fluid communication with said main chamber upper extremity, said tube further having a distal end capable of providing axial gas flow out of said tube.

Accordingly, it is desirable to provide an ostomy appliance configured to permit gas flow to a filter assembly in a desired manner, while substantially restricting flow of liquid, semisolids or solids to the filter while limiting or excluding additional material to do so.

### SUMMARY

The present disclosure provides an ostomy appliance as detailed in claim 1. Advantageous features are provided in dependent claims.

In an embodiment, the one or more flow elements may be a plurality of flow elements. The plurality of flow elements may be arranged in a staggered configuration. The plurality of flow elements may include a first plurality of flow elements and a second plurality of flow elements.

In an embodiment, the first plurality of flow elements may be arranged in a first row and the second plurality of flow elements may be arranged in a second row.

In an embodiment, a number of flow elements of the first plurality of flow elements may be different than a number of flow elements of the second plurality of flow elements.

In an embodiment, the plurality of flow elements may be formed in a predetermined shape. The first plurality of flow elements may have a first orientation and the second plurality of flow elements may have a second orientation different than the first orientation.

In an embodiment, the first plurality of flow elements may be substantially V-shaped and the second plurality of flow elements may be partially V-shaped.

In an embodiment, the first plurality of flow elements may have fixed ends at a first side of the filter flow path and free ends spaced from a second side of the filter flow path, and the second plurality of flow elements may have fixed ends at the second side of the filter flow path and free ends spaced from the first side of the filter flow path.

In an embodiment, flow elements of the first plurality of flow elements may be positioned alternately with flow elements of the second plurality of flow elements along the filter flow path. In an embodiment, the one or more flow elements are formed as heat seals.

In an embodiment, the outer wall may further include an embossed section having a plurality of embossed projections and channels between the projections. In an embodiment, one or more heat seals may be intermittently positioned along the embossed section. The one or more flow elements may include the projections. The projections may be diamond-shaped in the plan view.

In an embodiment, the ostomy appliance may also include a perforation extending between the filter flow path and the collection area. In an embodiment, the ostomy appliance may further include an inner wall formed by a heat seal between the first side and the second side, and at least a portion of the filter flow path may extend through a space between the inner wall and the outer periphery. In one embodiment, the filter flow path may include a first arm extending in different directions relation to one another. The filter assembly may include a first filter assembly disposed in the first arm and a second filter assembly disposed in the second arm.

Other objects, features, and advantages of the disclosure will be apparent from the following description, taken in conjunction with the accompanying sheets of drawings, wherein like numerals refer to like parts, elements, components, steps, and processes.

### BRIEF DESCRIPTION OF THE DRAWINGS

FIG. 1 is a plan diagram of an ostomy appliance according to an embodiment;
FIG. 2 is a plan diagram of an ostomy appliance according to another embodiment;
FIG. 3 is a plan diagram of an ostomy appliance according to another embodiment;
FIGS. 4A-4D are diagrams showing different examples of flow elements which may be provided at Detail B of FIG. 3;
FIG. 5 is a cross-sectional diagram taken at section A-A in FIG. 3;
FIG. 6 is a plan diagram of an ostomy appliance according to another embodiment; and
FIG. 7 is a plan diagram of an ostomy appliance according to another embodiment.
FIG. 8 is a plan diagram of an ostomy appliance according to another embodiment;
FIG. 9 is an enlarged plan view illustrating an example of an embossed section, according to an embodiment;
FIG. 10 is a cross-sectional diagram of an ostomy appliance schematically illustrating an example of an embossed section, according to an embodiment; and
FIG. 11 is a plan diagram of an ostomy appliance according to another embodiment.

### DETAILED DESCRIPTION

While the present disclosure is susceptible of embodiment in various forms, there is shown in the drawings and will hereinafter be described one or more embodiments with the understanding that the present disclosure is to be considered illustrative only and is not intended to limit the disclosure to any specific embodiment described or illustrated.

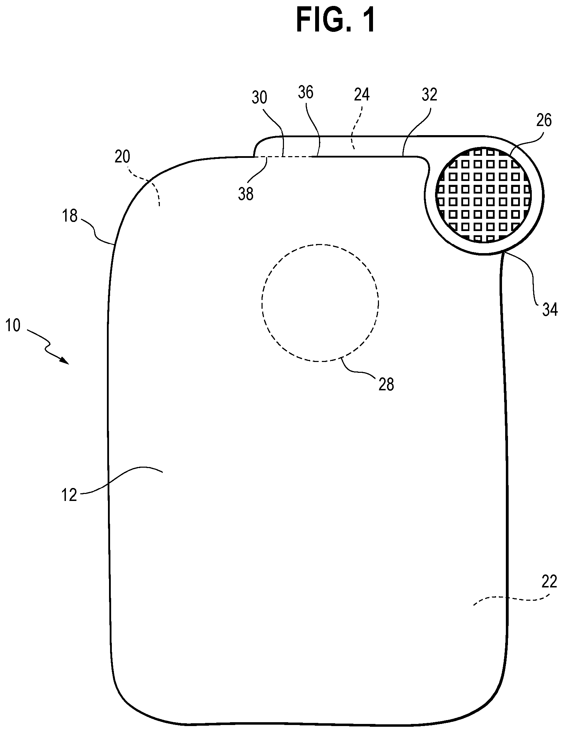

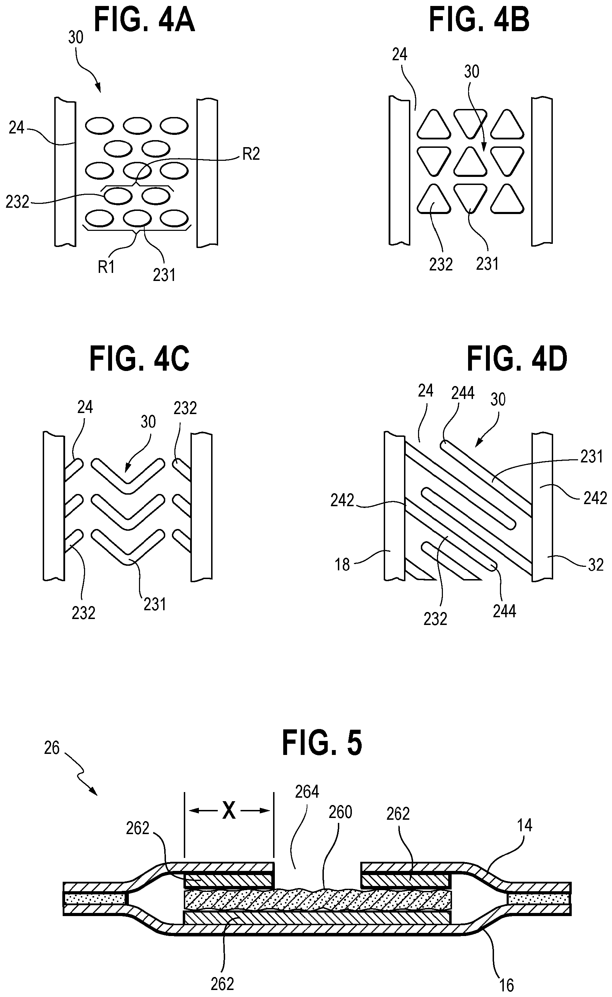

FIG. 1 is a plan diagram of an ostomy appliance 10, according to an embodiment. The ostomy appliance 10 may be an ostomy pouch. The ostomy appliance 10 includes an outer wall 12 having first and second opposed sides 14, 16 (see FIG. 5) joined together at an outer periphery 18. The outer wall 12 defines an interior volume 20, which according to one embodiment, may include a collection area 22 and a filter flow path 24 fluidically connected to the collection area 22. The ostomy appliance 10 also includes a filter assembly 26 fluidically connected to the collection area 22 by the filter flow path 24. An inlet 28 may be included in one of the first and second opposed sides 14, 16 to allow for ingress of contents, such as bodily waste, for receipt in the collection area 22.

The ostomy appliance 10 also includes one or more flow elements 30 disposed in the interior volume 20. The one or more flow elements 30 are formed as heat seals between the opposed first and second sides 14, 16. Thus, the one or more flow elements 30 are formed continuously with the outer wall 12 (and first and second sides 14, 16), and substantially fixed relative to the first and second sides 14, 16. The one or more flow elements 30 may be disposed at a boundary of the collection area 22 and the filter flow path 24, within the filter flow path 24, within the collection area 22, or combinations thereof. It will be appreciated that heat seals may include seals formed by, for example, plastic welding or Radio Frequency (RF) welding and/or other suitable techniques.

In one embodiment, the one or more flow elements 30 are a plurality of flow elements 30. Flow elements of the plurality of flow elements 30 may be spaced apart from one another by channels such that fluid, namely a gas, may flow through the channels between and around the flow elements 30. Accordingly, a gas may be vented from the collection area 22 into the filter flow path 24 and to the filter assembly 26 by flowing through channels between and around the flow elements 30. As described further below, the gas may then flow through the filter assembly 26 where it may be odor filtered, and to the atmosphere external of the outer wall 12. Thus, the flow elements 30 and corresponding channels may be sized and arranged so that gas venting is permitted through the channels (i.e., between and around the flow elements), but flow of liquids or solids past the flow elements 30 may be substantially restricted. Thus, characteristics of the flow elements 30, such as but not limited to a size, shape, position, spacing and/or orientation, may be varied during manufacture. Accordingly, a desired level of gas flow (venting) to the filter assembly 26 may be achieved while restricting solid, semisolid or liquid flow to the filter assembly 26 in a desired manner.

The ostomy appliance 10 may also include an inner wall 32. In one embodiment, the inner wall 32 may be formed as a heat seal between the first side 14 and the second side 16. That is, the inner wall 32 may be formed continuously with the outer wall 12. In one embodiment, at least a portion of the filter flow path 24 extends in a space between the inner wall 32 and the outer periphery 18. In one embodiment, the inner wall 32 may have a first end 34 joined to the outer periphery 18 and a second end 36 spaced from the outer periphery 18. The inner wall 32 may separate at least a portion of the filter flow path 24 from the collection area 22. In one embodiment, the filter flow path 24 is fluidically connected to the collection area 22 by a passage 38 adjacent to the second end 36. The passage 38 may extend between the second end 36 and a portion of the outer periphery 18. In one embodiment, the passage 38 may be positioned at or near a center, top portion of the ostomy appliance 10. Such positioning may provide desirable gas flow characteristics when the wearer of the ostomy appliance 10 is upright or laying down.

Accordingly, in the embodiments above, the filter flow path 24 may be a substantially tortuous flow path configured to allow gas flow from the collection area 22 to the filter assembly 26 while restricting or limiting liquid, semisolid and/or solid flow to the filter assembly 26. In one embodiment, the filter flow path 24 may be made tortuous by way of the one or more flow elements 30. Alternatively, or in addition, the filter flow path 24 may be defined, in part, between the inner wall 32 and a portion of the outer periphery 18, and the inner wall 32 may be shaped to provide a tortuous flow path 24.

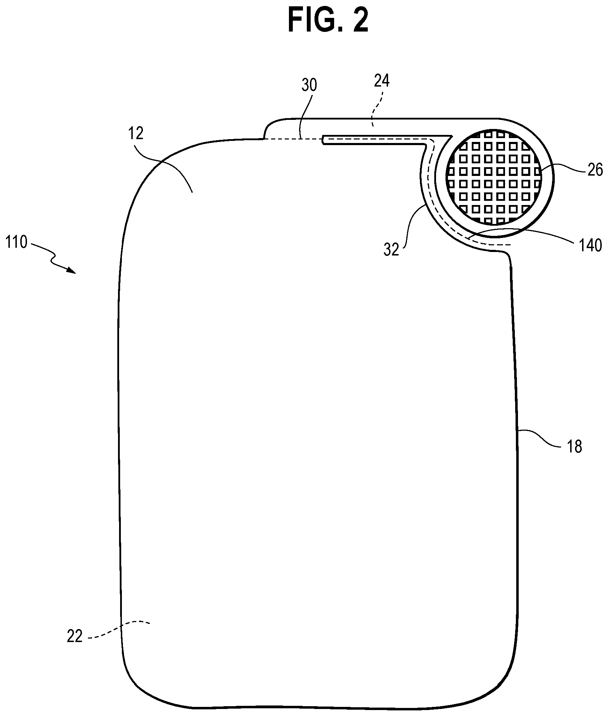

FIG. 2 is a plan diagram of an ostomy appliance 110 according to another embodiment. The ostomy appliance 110 may be substantially the same as the ostomy appliance described above with reference to FIG. 1, with the exception of any added, removed or different features described below. Accordingly, like features may be identified in the drawings with the same reference numbers as above, and further description of like features may be omitted below. In one embodiment, the ostomy appliance 110 includes a perforation 140 disposed between a portion of the filter flow path 24 and the collection area 22. The perforation 140 is configured to allow for a section of the outer wall 12 in which at least a portion of the filter flow path 24 extends to be separated from another section of the outer wall 12 in which the collection area 22 is disposed. Thus, by separating the ostomy appliance 10 at the perforation 140, the portion of the filter flow path 24 is allowed to "flop" or otherwise hang substantially freely from the section of the outer wall 12 in which the collection area 22 is formed. Such a free-hanging configuration is configured to add another aspect of tortuosity to the filter flow path 24. In one embodiment, the perforation 140 may be formed generally along the inner wall 32.

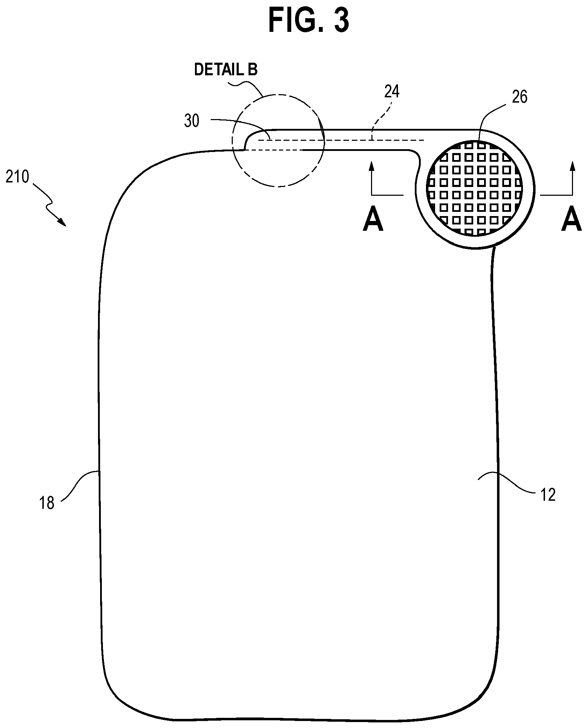

FIG. 3 is a plan diagram of an ostomy appliance 210 according to another embodiment. The ostomy appliance 210 may be substantially the same as the ostomy appliances described above with reference to FIGS. 1 and 2, with the exception of any added, removed and/or or different features described below. Accordingly, like features may be identified in the drawings with the same reference numbers as above, and further description of like features may be omitted below. In one embodiment, the flow elements 30 may be positioned within the filter flow path 24. Such positioning of the flow elements 30 may further restrict flow of liquid, semisolid or solid materials to the filter assembly 26 through the filter flow path 24. By positioning the flow elements 30 within the filter flow path, generally along a direction of flow toward the filter assembly 24, additional tortuosity may be provided to further restrict flow of unwanted materials (e.g., liquid, semisolid, solid materials) while still allowing for flow of gas to the filter assembly 26.

FIGS. 4A-4D are enlarged views of Detail B in FIG. 3, showing different examples of flow elements 30. In one embodiment, the one or more flow elements 30 may include a plurality of flow elements 30. Flow elements of the plurality of flow elements 30 may be staggered relative to one another. For example, flow elements 30 may be disposed generally along a direction of flow in the filter flow path 24 and staggered relative to one another in a lateral direction relative to the direction of flow.

With reference to FIG. 4A, in one embodiment, the plurality of flow elements 30 include a first plurality 231 of flow elements and a second plurality 232 of flow elements 30. The first plurality 231 of flow elements 30 may be arranged in a first row R1 and the second plurality 232 of flow elements 30 may arranged in a second row R2. In one embodiment, a number of flow elements 30 in the first plurality 231 of flow elements is different than a number of flow elements 30 in the second plurality of flow elements 231. In one embodiment, a number of flow elements 30 in the first row R1 is different than a number of flow elements 30 in the second row R2.

In one embodiment, the plurality of flow elements 30 are formed in a predetermined shape. As non-limiting examples, the flow elements may be substantially circular, oval, elliptical, or polygonal shapes such as triangular, square, rectangular and the like. In some embodiments, the flow elements 30 may be formed as elongated members, such as a line segment, a curve segment, a plurality of line segments, a plurality of curve segments, and the like.

In one embodiment, the first plurality 231 of flow elements 30 may have a first orientation and the second plurality 232 of flow elements 30 have a second orientation. In one embodiment, the first orientation may be different than the second orientation. For example, with reference to FIG. 4B, the flow elements 30 may be substantially triangular in shape. The first plurality 231 of flow elements may have a first orientation and the second plurality 232 of flow elements may have a second orientation different than the first. For example, the second plurality 232 of flow elements 30. may be rotated a predetermined amount, such as 180 degrees, relative to the first plurality 231 of flow elements 30.

With reference to FIG. 4C, in one embodiment, the first plurality 231 of flow elements 30 may be substantially V-shaped, i.e., formed by a plurality of line segments disposed at an angle relative to one another. The second plurality 232 of flow elements 30 may be formed as partially in V-shape, i.e., as line segments. In one embodiment, flow elements 30 of the second plurality 232 may be disposed at an acute angle relative to either the outer periphery 18 and/or inner wall 32. It is understood, however, that the present disclosure is not limited to line segments or the "V-shaped" configuration above. For example, curved segments and other shapes, such as C-shapes or U-shapes are envisioned as well. A zig-zag configuration is also envisioned.

Referring to FIG. 4D, in one embodiment, the first plurality 231 of flow elements 30 have fixed ends 242 at a first side of the filter flow path 24 and free ends 244 spaced from a second side of the filter flow path 24. The second plurality 232 of flow elements 30 may have fixed ends 242 at the second side of the filter flow path 24 and free ends 244 spaced from the first side of the filter flow path 24. In one embodiment, the first side of the filter flow path 24 may be formed by the inner wall 32 and the second side may be formed by the outer periphery 18. In one embodiment, flow elements 30 of the first plurality 231 of flow elements 30 are positioned alternately with flow elements 30 of the second plurality 231 of flow elements 30 along the filter flow path 24. In one embodiment, the flow elements 30 may be substantially parallel to one another but are not limited to such a configuration.

FIG. 5 is a cross-sectional section view of the filter assembly 26 taken at section A-A in FIG. 3, according to an embodiment. The filter assembly 26 may include a filter media 260, such as a charcoal, carbon or other suitable media for deodorizing gas passing therethrough. The filter assembly 26 may also include a sealing material 262 disposed over portions of the filter media 260. The sealing material 262 may be substantially gas impermeable such that gas received from the collection area 22 via the filter flow path 24 must flow around the sealing material 262 to enter and exit the filter media 260. Thus, the sealing material 262 may also provide tortuosity along the gas flow path. In one embodiment, the filter assembly 26 may be a radial flow filter such that gas flows through the filter media in at least a radial direction of the media 260.

In one embodiment, the filter assembly 26 is sealed between the first and second sides 14, 16 of the outer wall 12, for example, with the sealing material 262. In one embodiment, the sealing material is that of the first and second sides 14, 16, i.e., the filter assembly 26 may be sealed directly to one or both of the first and second sides 14, 16. One of the first side 14 and second side 16 includes an outlet 264 configured to dispose the filter assembly 26, and in particular the filter media 260 in fluid communication with the external atmosphere for egress of gas from the ostomy appliance 10 after filtering. In some embodiments, the filter assembly 26 may include a membrane, such as a hydrophobic or oleophobic membrane extending across at least a portion of the filter media 260.

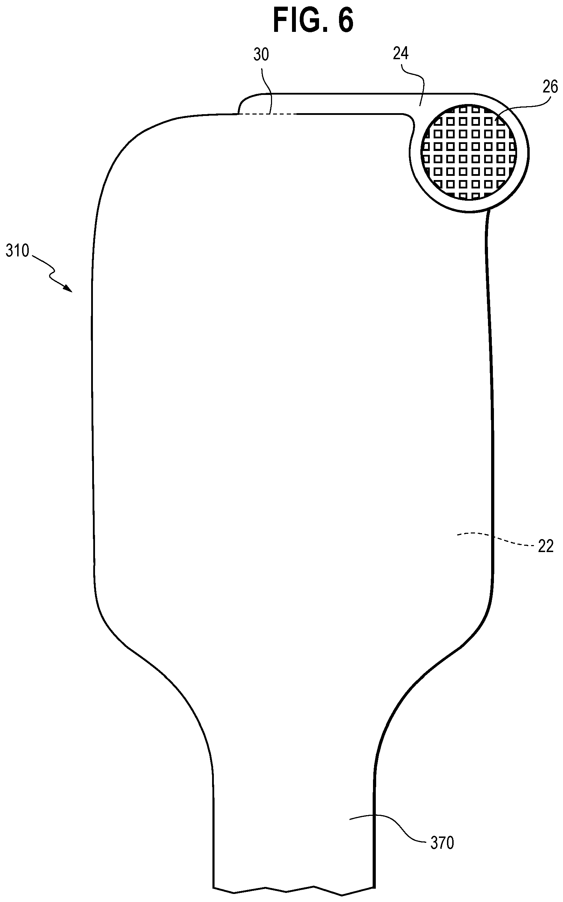

FIG. 6 is a plan diagram of an ostomy appliance 310 according to another embodiment. The ostomy appliance 310 may be substantially the same as any of the ostomy appliances described above with the exception of any added, removed and/or different features described below. Accordingly, like features may be identified in the drawings with the same reference numbers as above, and further description of like features may be omitted below. In one embodiment, the ostomy appliance 310 may be a drainable ostomy pouch. Thus, the ostomy appliance 310 may be formed having a drainage outlet 370 at one end. In one embodiment, the drainage outlet 370 is movable from a closed position in which the ostomy appliance 310 is configured to collect and store bodily waste in the collection area 22, and a drainage position, shown in FIG. 6, in which the bodily waste may be drained from the collection area 22.

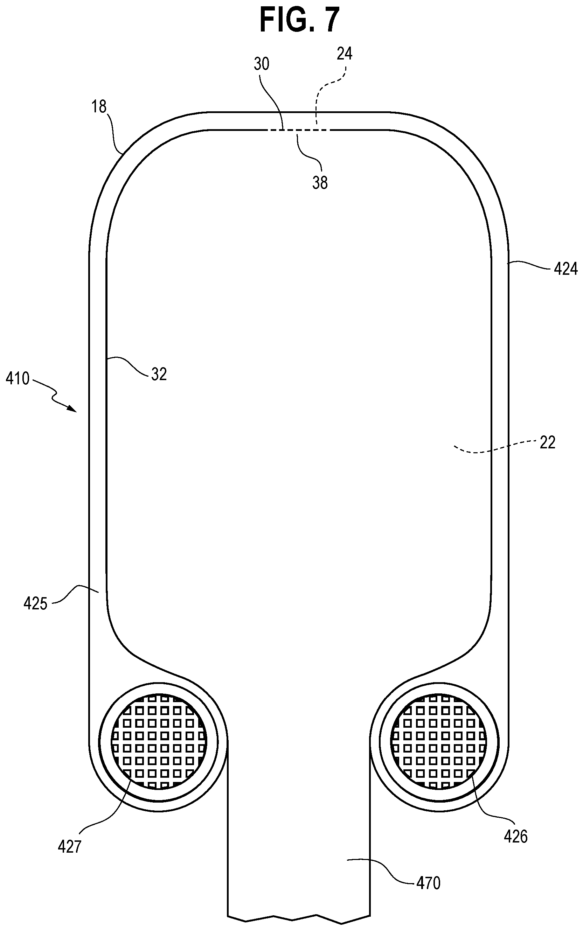

FIG. 7 is a plan diagram of an ostomy appliance 410 according to another embodiment. The ostomy appliance 410 may be substantially the same as any of the ostomy appliances described above with the exception of any added, removed and/or different features described below. Accordingly, like features may be identified in the drawings with the same reference numbers as above, and further description of like features may be omitted below.

In one embodiment, the ostomy appliance 410 includes the filter flow path 24, and the filter flow path 24 may include a first arm 424 and a second arm 425. The first arm 424 and the second arm 425 may extend in generally opposite directions relative to one another from the passage 38. The filter assembly may include a first filter assembly 426 disposed in the first arm 424 and a second filter assembly 427 disposed in the second arm 425. In one embodiment, the ostomy appliance 410 may include a drainage outlet 470. In one embodiment, the passage 38 may be disposed at one end 472, such as an upper end, of the ostomy appliance and the drainage outlet 470 may be disposed at another end 474, such as a lower end, of the ostomy appliance 410, opposite to the one end 472.

In one embodiment, the first and second filter assemblies 426, 427 may be disposed at or near respective ends of the first and second arms 424, 425 opposite to the passage 38. In one embodiment, the drainage outlet 470 may extend between the first and second filter assemblies 426, 427. In one embodiment, first and second perforations (not shown) may extend between at least a portion of the first and second arms 424, 425 and the collection area 22 or drainage outlet 470 of the ostomy appliance 410. Thus, the first and second arms 424, 425 may be separated from a portion of the appliance 410, such as along a portion of the inner wall 32, to hang freely from the inner wall 32, thereby adding to the tortuosity of the filter path 24.

Accordingly, in the embodiments above, the ostomy appliance may be formed, for example, by an additional heat seal step (or steps) to provide the one or more flow elements 30, the inner wall 32, or both, without adding material to an existing ostomy pouch. The perforations 140, may be formed, for example, by stamping, punching, cutting or the like. In addition, more precise gas flow control characteristics may be provided by the one or more flow elements 30, for example, by controlling the shape, orientation, positioning, size, or combinations thereof, during manufacture. In one embodiment, a gas channel between adjacent flow elements 30 may be opened in response to a build up of gas pressure in the collection area 22.

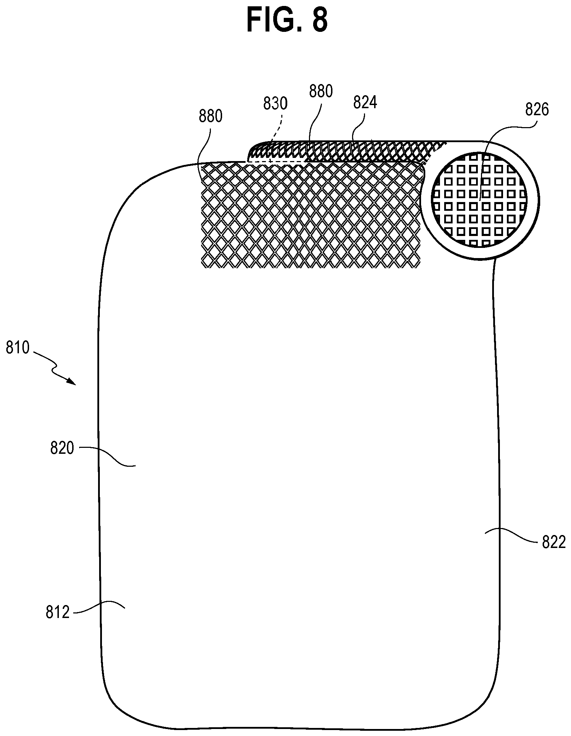

FIG. 8 is a plan diagram of an ostomy appliance 810 according to another embodiment. Some features of the ostomy appliance 810 may be the same or substantially the same as features of the ostomy appliances discussed in embodiments above. Further description of such features may be omitted below.

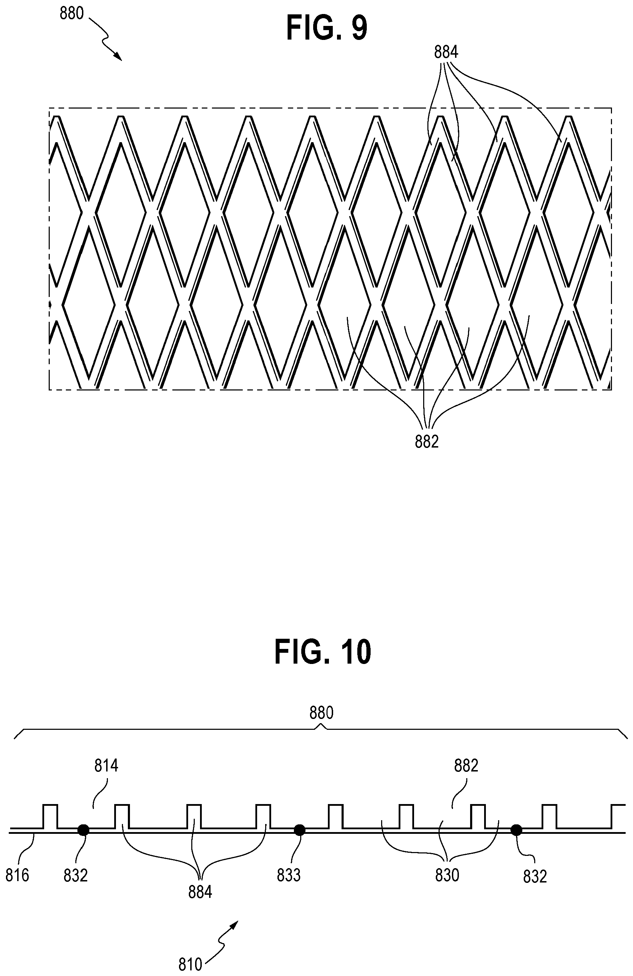

The ostomy appliance 810 may generally include an outer wall 812 having first and second sides 814, 816 (FIG. 10) and define an interior volume 820 having a collection area 822. The ostomy appliance 810 may also include a filter flow path 824 and a filter assembly 826 similar to those described in the embodiments above.

The ostomy appliance 810 may also include one or more embossed sections 880. The one or more embossed sections 880 may be disposed upstream from the filter assembly 826. For example, the one or more embossed sections 880 may extend along either of the filter flow path 824, the collection area 822, or both. In an embodiment, the one or more embossed sections 880 extends completely across a passage 830 from the collection area 822 to the filter flow path 824. In this manner, the one or more embossed sections 880 are positioned along the flow path of gas being vented from the collection area 822 to the filter assembly 826.

FIG. 9 is an enlarged plan view illustrating an example of an embossed section 880, according to an embodiment. The embossed section 880 may include a plurality of embossed shapes 882 and channels 884 between the embossed shapes 882. In an embodiment, the embossed shapes 882 are a plurality of embossed diamond shapes and channels extending between the embossed diamond shapes. However, the embossed section 880 may include other suitable embossed shapes, including combinations of different shapes.

FIG. 10 is a cross-sectional diagram of the ostomy appliance 810 schematically illustrating an example of an embossed section 880, according to an embodiment. In an embodiment, the embossed section 880 may be formed in or on the first side 814, the second side 816, or both, of the outer wall 812. For example, as shown in the example of FIG. 10, the embossed section 880 may be an embossed section of the first side 814. In an embodiment, the first side 814 and/or the second side 816 may be a known film suitable for use in ostomy appliances. Accordingly, the embossed section 880 may be a section of an embossed film.

Alternatively, an embossed film may be connected to the outer wall 812 to form the embossed section 880. The embossed film may be disposed in the interior volume 820. The embossed film may be connected to one of the first side 814 and the second side 816.

The embossed shapes 882 may form inwardly directed projections 830 separated by channels 884. In an embodiment, one or more of the projections 830 may be heat sealed or otherwise fastened to an opposite side of the ostomy appliance 810. For example, in an embodiment, a heat seal 832 may be formed at one or more of the projections 830 to fasten the embossed section 880 to the second side 816. In an embodiment, a plurality of heat seals 832 may be intermittently positioned on the embossed section 880 to fasten the embossed section 880 to the opposite side of the ostomy appliance 810. A hear seal 832 and a projection 830 may be formed continuously with another, i.e., without the addition of extra materials.

Accordingly, flow elements may be formed by each of the projections 830 and/or by combinations of the projections 830 and heat seals 832. The heat seals 832 may be configured to limit a distance the first side 814 may move away from the second side 816, and vice versa, for example, in response to a gas pressure increase within the ostomy appliance 810.

The channels 884 extend between the projections 830. The projections 830 and channels 884 may be sized and arranged such that gas from the collection area 822 may flow in the channels 884 between and around flow elements, i.e., the projections 830 and heat seals 832, to the filter assembly 826. Further, the projections 830 and channels 884 may be sized and arranged such that flow of liquid and/or solids through the channels 884 and the projections 830 is substantially restricted or prevented. Further still, and as described above, movement of the projections 830 away from the opposite side (e.g., the second side 816 in FIG. 10) may be restricted by the heat seals 832. In an embodiment, movement of the projections 830 away from the opposite side may be limited to an extent where gas flow between the projections 830 and the opposite side may be permitted, but liquid and/or solid flow is substantially restricted. Thus, a filter path may be formed by one or more embossed sections 880 upstream from the filter assembly 826.

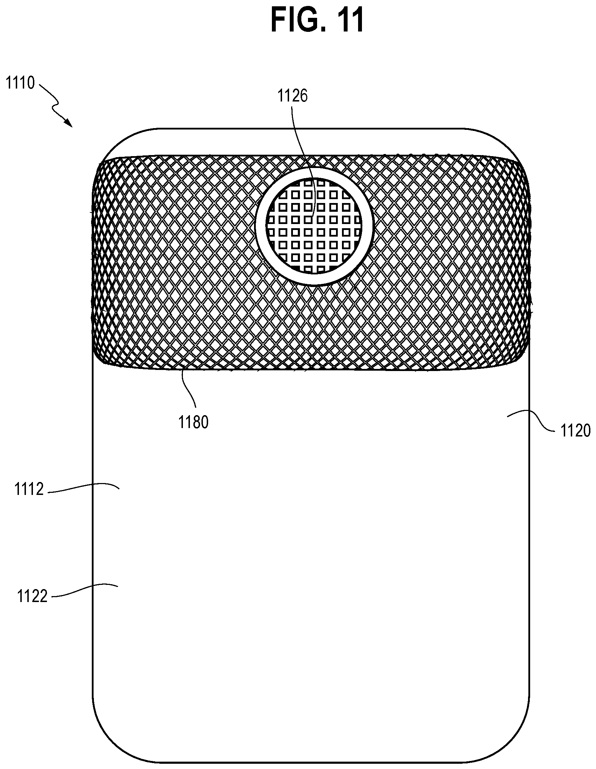

FIG. 11 is a plan diagram of an ostomy appliance 1110 according to another embodiment. Some features of the ostomy appliance 1110 may be the same or substantially the same as features of the ostomy appliances discussed in embodiments above. Further description of such features may be omitted below.

The ostomy appliance 1110 may include an embossed section 1180 and a filter assembly 1126. The filter assembly 1126 may be positioned such that gas vented from the collection area 1122 flow through the embossed section 1180 before reaching the filter assembly 1126. In an embodiment, the filter assembly 1126 may be surrounded or substantially surrounded by the embossed section 1180. In an embodiment, the filter assembly 1126 may be disposed in the embossed section 1180. The embossed section 1180 may include the features of the embossed section 880 described above and shown, for example, in FIGS. 9 and 10. A filter path may be formed by the embossed section 1180 in the interior volume 1120 of the ostomy appliance 1110 adjacent to the collection area 1122. The ostomy appliance 1110 may include an outer wall having opposed first and second sides as described in embodiments above, and shown, for example, in FIG. 10 at 814 and 816. The embossed section 1180 may be formed in the outer wall 1112 at one or both of the first side and the second side of the outer wall 1112 and include a plurality of heat seals to attach the opposing sides of the outer wall 1112.

It is understood that the relative directions described above, e.g., "upward," "downward," "upper," "lower," "above," "below," are used for illustrative purposes only and may change depending on an orientation of the ostomy pouch and/or the patient. Accordingly, this terminology is non-limiting in nature. In addition, it is understood that one or more various features of an embodiment above may be used in, combined with, or replace other features of a different embodiment described herein.

In the present disclosure, the words "a" or "an" are to be taken to include both the singular and the plural. Conversely, any reference to plural items shall, where appropriate, include the singular.

## Claims

1. An ostomy appliance (10, 110, 210, 310, 410, 810, 1110) comprising:
an outer wall (12) having first and second opposed sides (14, 16) joined at an outer periphery (18), an inlet and a filter assembly (26), wherein the outer wall defines an interior volume (20) comprising a collection area (22) and a filter flow path (24) in fluid communication with the collection area, the filter flow path fluidically connecting the filter assembly to the collection area, and
an inner wall (32) formed between the first and second opposed sides (14, 16), wherein the inner wall (32) separates at least a portion of the filter flow path (24) from the collection area (22),
the inner wall (32) forms a first side of the filter flow path (24) and the outer periphery (18) forms a second side of the filter flow path (24),
one or more flow elements (30, 231, 232) comprising one or more flow elements disposed at a boundary of the collection area (22) and the filter flow path (24), **characterised in that**
the one or more flow elements further comprising a first plurality of flow elements (231) and a second plurality of flow elements (232), the first and second plurality of flow elements being formed continuously with the outer wall and between the first side and the second side,
the first plurality of flow elements (231) having fixed ends (242) at the first side of the filter flow path and free ends (244) spaced from the second side of the filter flow path (24), and the second plurality of flow elements (232) having fixed ends (242) at the second side of the filter flow path and free ends (244) spaced from the first side of the filter flow path (24), and **in that** the first plurality of flow elements and second plurality of flow elements are sized and arranged to permit gas venting from the collection area to the filter assembly and restrict liquid and/or solid flow from the collection area to the filter assembly.

2. The ostomy appliance (10, 110, 210, 310, 410, 810, 1110) of claim 1, wherein the one or more flow elements comprise a third plurality of flow elements (30) arranged in a staggered configuration.

3. The ostomy appliance (10, 110, 210, 310, 410, 810, 1110) of claim 1, wherein a number of flow elements of the first plurality of flow elements is different than a number of flow elements of the second plurality of flow elements.

4. The ostomy appliance (10, 110, 210, 310, 410, 810, 1110) of any preceding claim, wherein the first plurality of flow elements (231) have a first orientation, and the second plurality of flow elements (232) have a second orientation, wherein the first orientation is different than the second orientation.

5. The ostomy appliance (10, 110, 210, 310, 410, 810, 1110) of any preceding claim, wherein flow elements of the first plurality of flow elements are positioned alternately with flow elements of the second plurality of flow elements (232) along the filter flow path.

6. The ostomy appliance (10, 110, 210, 310, 410, 810, 1110) of any of preceding claim, wherein the one or more flow elements (30) are formed as heat seals.

7. The ostomy appliance of (10, 110, 210, 310, 410, 810, 1110) of claim 1, the outer wall further comprising an embossed section (880) having a plurality of embossed projections and channels between the plurality of embossed projections, and one or more heat seals (832) intermittently positioned along the embossed section.

8. The ostomy appliance (10, 110, 210, 310, 410, 810, 1110) of claim 7, wherein the one or more flow elements of the third plurality of flow elements (30) include the projections of the plurality of embossed projections (830).

9. The ostomy appliance (10, 110, 210, 410, 810, 1110) of any of claims 7 or 8, wherein the projections (830) are diamond- shaped in a plan view.

10. The ostomy appliance (10, 110, 210, 310, 410, 810, 1110) of any preceding claim, further comprising a perforation (140) extending between the filter flow path and the collection area.

11. The ostomy appliance (10, 110, 210, 310, 410, 810, 1110) of any preceding claim, wherein the inner wall (32) is formed by a heat seal between the first side and the second side, wherein at least a portion of the filter flow path (24) extends through a space between the inner wall and the outer periphery.

12. The ostomy appliance (10, 110, 210, 310, 410, 810, 1110) of any preceding claim, wherein the filter flow path (24) includes a first arm (424) facilitating a first flow direction and a second arm (425) facilitating a second flow direction, different from the first flow direction, and wherein the filter assembly includes a first filter assembly (426) disposed in the first arm and a second filter assembly (427) disposed in the second arm.

## Patentansprüche

1. Stomavorrichtung (10, 110, 210, 310, 410, 810, 1110), umfassend:
eine Außenwand (12) mit einer ersten und einer zweiten gegenüberliegenden Seite (14, 16), die an einem Außenumfang (18) verbunden sind, einem Einlass und einer Filteranordnung (26), wobei die Außenwand ein Innenvolumen (20) definiert, das einen Sammelbereich (22) und einen Filterströmungspfad (24) umfasst, der in Fluidverbindung mit dem Sammelbereich steht, wobei der Filterströmungspfad die Filteranordnung fluidisch mit dem Sammelbereich verbindet, und
eine Innenwand (32), die zwischen der ersten und der zweiten gegenüberliegenden Seite (14, 16) ausgebildet ist, wobei die Innenwand (32) zumindest einen Abschnitt des Filterströmungspfads (24) von dem Sammelbereich (22) trennt,
wobei die Innenwand (32) eine erste Seite des Filterströmungspfads (24) ausbildet und der Außenumfang (18) eine zweite Seite des Filterströmungspfads (24) ausbildet,
eines oder mehrere Strömungselemente (30, 231, 232), umfassend eines oder mehrere Strömungselemente, die an einer Grenze des Sammelbereichs (22) und des Filterströmungspfads (24) angeordnet sind, **dadurch gekennzeichnet, dass**
das eine oder die mehreren Strömungselemente des Weiteren eine erste Vielzahl von Strömungselementen (231) und eine zweite Vielzahl von Strömungselementen (232) umfassen, wobei die erste und die zweite Vielzahl von Strömungselementen durchgehend mit der Außenwand und zwischen der ersten Seite und der zweiten Seite ausgebildet sind,
wobei die erste Vielzahl von Strömungselementen (231) fixierte Enden (242) an der ersten Seite des Filterströmungspfads und freie Enden (244) aufweist, die von der zweiten Seite des Filterströmungspfads (24) beabstandet sind, und die zweite Vielzahl von Strömungselementen (232) fixierte Enden (242) an der zweiten Seite des Filterströmungspfads und freie Enden (244) aufweist, die von der ersten Seite des Filterströmungspfads (24) beabstandet sind, und dass die erste Vielzahl von Strömungselementen und die zweite Vielzahl von Strömungselementen so bemessen und angeordnet sind, dass sie ein Entweichen von Gas aus dem Sammelbereich zur Filteranordnung zulassen und einen Fluss von Flüssigkeit und/oder Feststoffen aus dem Sammelbereich zur Filteranordnung einschränken.

2. Stomavorrichtung (10, 110, 210, 310, 410, 810, 1110) nach Anspruch 1, wobei das eine oder die mehreren Strömungselemente eine dritte Vielzahl von Strömungselementen (30) umfassen, die in einer versetzten Konfiguration angeordnet sind.

3. Stomavorrichtung (10, 110, 210, 310, 410, 810, 1110) nach Anspruch 1, wobei eine Anzahl von Strömungselementen der ersten Vielzahl von Strömungselementen sich von einer Anzahl von Strömungselementen der zweiten Vielzahl von Strömungselementen unterscheidet.

4. Stomavorrichtung (10, 110, 210, 310, 410, 810, 1110) nach einem der vorhergehenden Ansprüche, wobei die erste Vielzahl von Strömungselementen (231) eine erste Ausrichtung aufweist und die zweite Vielzahl von Strömungselementen (232) eine zweite Ausrichtung aufweist, wobei sich die erste Ausrichtung von der zweiten Ausrichtung unterscheidet.

5. Stomavorrichtung (10, 110, 210, 310, 410, 810, 1110) nach einem der vorhergehenden Ansprüche, wobei Strömungselemente der ersten Vielzahl von Strömungselementen abwechselnd mit Strömungselementen der zweiten Vielzahl von Strömungselementen (232) entlang des Filterströmungspfads positioniert sind.

6. Stomavorrichtung (10, 110, 210, 310, 410, 810, 1110) nach einem der vorhergehenden Ansprüche, wobei das eine oder die mehreren Strömungselemente (30) als Heißsiegelungen ausgebildet sind.

7. Stomavorrichtung (10, 110, 210, 310, 410, 810, 1110) nach Anspruch 1, wobei die Außenwand des Weiteren einen geprägten Teilabschnitt (880) umfasst, der eine Vielzahl von geprägten Vorsprüngen und Kanälen zwischen der Vielzahl von geprägten Vorsprüngen aufweist, und eine oder mehrere Heißsiegelungen (832), die intermittierend entlang des geprägten Teilabschnitts positioniert sind.

8. Stomavorrichtung (10, 110, 210, 310, 410, 810, 1110) nach Anspruch 7, wobei das eine oder die mehreren Strömungselemente der dritten Vielzahl von Strömungselementen (30) die Vorsprünge der Vielzahl von geprägten Vorsprüngen (830) einschließen.

9. Stomavorrichtung (10, 110, 210, 410, 810, 1110) nach einem der Ansprüche 7 oder 8, wobei die Vorsprünge (830) in einer Draufsicht rautenförmig sind.

10. Stomavorrichtung (10, 110, 210, 310, 410, 810, 1110) nach einem der vorhergehenden Ansprüche, des Weiteren umfassend eine Perforation (140), die sich zwischen dem Filterströmungspfad und dem Sammelbereich erstreckt.

11. Stomavorrichtung (10, 110, 210, 310, 410, 810, 1110) nach einem der vorhergehenden Ansprüche, wobei die Innenwand (32) durch eine Heißsiegelung zwischen der ersten Seite und der zweiten Seite ausgebildet ist, wobei sich zumindest ein Abschnitt des Filterströmungspfads (24) durch einen Raum zwischen der Innenwand und dem Außenumfang erstreckt.

12. Stomavorrichtung (10, 110, 210, 310, 410, 810, 1110) nach einem der vorhergehenden Ansprüche, wobei der Filterströmungspfad (24) einen ersten Arm (424), der eine erste Strömungsrichtung ermöglicht, und einen zweiten Arm (425) einschließt, der eine zweite Strömungsrichtung ermöglicht, die sich von der ersten Strömungsrichtung unterscheidet, und wobei die Filteranordnung eine erste Filteranordnung (426), die im ersten Arm angeordnet ist, und eine zweite Filteranordnung (427) einschließt, die im zweiten Arm angeordnet ist.

## Revendications

1. Appareil de stomie (10, 110, 210, 310, 410, 810, 1110) comprenant :
une paroi extérieure (12) ayant des premier et second côtés opposés (14, 16) reliés à une périphérie extérieure (18), une entrée et un ensemble de filtre (26), dans lequel la paroi extérieure définit un volume intérieur (20) comprenant une zone de collecte (22) et un trajet d'écoulement de filtre (24) en communication fluidique avec la zone de collecte, le trajet d'écoulement de filtre connectant fluidiquement l'ensemble de filtre à la zone de collecte, et
une paroi intérieure (32) formée entre les premier et second côtés opposés (14, 16), dans lequel la paroi intérieure (32) sépare au moins une partie du trajet d'écoulement de filtre (24) de la zone de collecte (22),
la paroi intérieure (32) forme un premier côté du trajet d'écoulement de filtre (24) et la périphérie extérieure (18) forme un second côté du trajet d'écoulement de filtre (24),
un ou plusieurs éléments d'écoulement (30, 231, 232) comprenant un ou plusieurs éléments d'écoulement disposés à une limite de la zone de collecte (22) et du trajet d'écoulement de filtre (24), **caractérisés en ce que**
les un ou plusieurs éléments d'écoulement comprennent également une première pluralité d'éléments d'écoulement (231) et une deuxième pluralité d'éléments d'écoulement (232), la première et la deuxième pluralité d'éléments d'écoulement étant formées en continu avec la paroi extérieure et entre le premier côté et le second côté,
la première pluralité d'éléments d'écoulement (231) ayant des extrémités fixes (242) du premier côté du trajet d'écoulement de filtre et des extrémités libres (244) espacées du second côté du trajet d'écoulement de filtre (24), et la deuxième pluralité d'éléments d'écoulement (232) ayant des extrémités fixes (242) du second côté du trajet d'écoulement de filtre et des extrémités libres (244) espacées du premier côté du trajet d'écoulement de filtre (24), et **en ce que** la première pluralité d'éléments d'écoulement et la deuxième pluralité d'éléments d'écoulement sont dimensionnées et disposées de manière à permettre l'évacuation des gaz de la zone de collecte vers l'ensemble de filtre et à limiter l'écoulement de liquide et/ou de solide de la zone de collecte vers l'ensemble de filtre.

2. Appareil de stomie (10, 110, 210, 310, 410, 810, 1110) selon la revendication 1, dans lequel les un ou plusieurs éléments d'écoulement comprennent une troisième pluralité d'éléments d'écoulement (30) disposés en configuration décalée.

3. Appareil de stomie (10, 110, 210, 310, 410, 810, 1110) selon la revendication 1, dans lequel un nombre d'éléments d'écoulement de la première pluralité d'éléments d'écoulement est différent d'un nombre d'éléments d'écoulement de la deuxième pluralité d'éléments d'écoulement.

4. Appareil de stomie (10, 110, 210, 310, 410, 810, 1110) selon une quelconque revendication précédente, dans lequel la première pluralité d'éléments d'écoulement (231) ont une première orientation, et la deuxième pluralité d'éléments d'écoulement (232) ont une seconde orientation, dans lequel la première orientation est différente de la seconde orientation.

5. Appareil de stomie (10, 110, 210, 310, 410, 810, 1110) selon une quelconque revendication précédente, dans lequel des éléments d'écoulement de la première pluralité d'éléments d'écoulement sont positionnés alternativement de la deuxième pluralité d'éléments d'écoulement (232) le long du trajet d'écoulement de fluide.

6. Appareil de stomie (10, 110, 210, 310, 410, 810, 1110) selon une quelconque revendication précédente, dans lequel les un ou plusieurs éléments d'écoulement (30) sont formés comme des joints thermiques.

7. Appareil de stomie (10, 110, 210, 310, 410, 810, 1110) selon la revendication 1, la paroi extérieure comprenant également une section gaufrée (880) ayant une pluralité de projections gaufrées et des canaux entre la pluralité de projections gaufrées, et un ou plusieurs joints thermiques (832) positionnés de manière intermittente le long de la section gaufrée.

8. Appareil de stomie (10, 110, 210, 310, 410, 810, 1110) selon la revendication 7, dans lequel les un ou plusieurs éléments d'écoulement de la troisième pluralité d'éléments d'écoulement (30) comportent les projections de la pluralité de projections gaufrées (830).

9. Appareil de stomie (10, 110, 210, 410, 810, 1110) selon l'une quelconque des revendications 7 ou 8, dans lequel les projections (830) sont en forme de losange en vue de dessus.

10. Appareil de stomie (10, 110, 210, 310, 410, 810, 1110) selon une quelconque revendication précédente, comprenant également une perforation (140) se prolongeant entre le trajet d'écoulement de filtre et la zone de collecte.

11. Appareil de stomie (10, 110, 210, 310, 410, 810, 1110) selon une quelconque revendication précédente, dans lequel la paroi intérieure (32) est formée par un joint thermique entre le premier côté et le second côté, dans lequel au moins une partie du trajet d'écoulement de filtre (24) se prolonge à travers un espace entre la paroi intérieure et la périphérie extérieure.

12. Appareil de stomie (10, 110, 210, 310, 410, 810, 1110) selon une quelconque revendication précédente, dans lequel le trajet d'écoulement de filtre (24) comporte un premier bras (424) facilitant une première direction d'écoulement et un second bras (425) facilitant une seconde direction d'écoulement, différente de la première direction d'écoulement, et dans lequel l'ensemble de filtre comporte un premier ensemble de filtre (426) disposé dans le premier bras et un second ensemble de filtre (427) disposé dans le second bras.
